# EUROPEAN PATENT SPECIFICATION

(11) **EP 1 685 402 B1**
(45) Date of publication and mention of the grant of the patent: **22.07.2009**
(21) Application number: 04810571.2
(22) Date of filing: 09.11.2004
(51) Int. Cl.: G01N 33/50, C07K 14/47

(54) **HAIRLESS PROTEIN-INTERACTING PARTNER COMPLEXES AND METHODS THEREOF FOR THE BEAUTIFICATION AND/OR IMPROVEMENT OF MAMMALIAN SKIN**
HAIRLESS-PROTEIN-INTERAGIERENDER PARTNER KOMPLEX UND METHODEN ZUR VERSCHÖNERUNG UND/ODER VERBESSERUNG VON SÄUGETIERHAUT
COMPLEXES HOMOLOGUES A INTERACTION AVEC LA PROTEINE SANS POIL ET METHODES ASSOCIEES D'EMBELLISSEMENT ET/OU D'AMELIORATION DE LA PEAU

(30) Priority: 13.11.2003 US 712629
(43) Date of publication of application: 02.08.2006
(73) Proprietor: THE PROCTER & GAMBLE COMPANY, Cincinnati, Ohio 45202 (US)
(72) Inventor: SREEKRISHNA, Kotikanyadanam, Cincinnati, Ohio 45242 (US); LEBOEUF, Robert, Alexander, Cincinnati, Ohio 45242 (US)
(74) Representative: Hampton, Matthew John
(86) International application number: PCT/US2004/037263
(87) International publication number: WO 2005/050201

(56) References cited:
- WO-A-99/38965
- US-B1- 6 348 348
- DJABALI K ET AL: "Interaction of the hairless protein with the thyroid hormone receptors" JOURNAL OF INVESTIGATIVE DERMATOLOGY, vol. 114, no. 4, April 2000 (2000-04), page 823, XP008045390 & 61ST ANNUAL MEETING OF THE SOCIETY FOR INVESTIGATIVE DERMATOLOGY.; CHICAGO, ILLINOIS, USA; MAY 10-14, 2000 ISSN: 0022-202X

## Description

### Field of Invention

The present invention relates to hairless protein-interacting partner complexes, antagonist or agonist active compounds, compositions and products comprising same and methods thereof. The complexes are useful for screening test agents and identifying compounds that convey material beautification and improvement benefits to mammalian, and particularly human, skin.

### Background of the Invention

Humans continue to demonstrate an obsession with appearance, particularly of the face, but increasingly of the skin and body as well. Indeed, many believe that appearance is intrinsically linked to self-esteem, the selection of a significant other, professional advancement and overall societal acceptance. The condition of human skin typically peaks at the age of 20, with subsequent thinning and loss of elasticity. Not surprisingly, American consumers spent approximately $2.8 billion in 2001 on products that claimed to slow or reverse the aging of skin. The demand for appearance-enhancing modalities continues to increase, as evidenced by the advent of many new products and services, each of which purports to achieve a desired appearance-enhancing result.

Lotions (herbal and otherwise), claiming a variety of skin benefits, are introduced to the skin care market globally every year. Other products having well-defined chemical entities (*e*.*g*., alpha-hydroxy acids, niacinamide, retinoids, vitamin E, kinetin, Copper-peptide) are also widely available. Despite the commercialization of such products, however, there remains a significant and unmet need for compositions and products that are useful in the prevention and reversal of skin aging. Indeed, various clinical and/or surgical procedures, including injections (*e*.*g.*, botulin toxin, collagen, hyaluronic acid, keratin, silicone, cadaver skin extract) have proven to be effective in the beautification of mammalian skin. Nevertheless, such procedures tend to be expensive and time consuming, and thus, generally unavailable to most consumers. Further, as with other medical procedures, clinical and surgical approaches to the beautification of mammalian skin are associated with many risks and side effects. Herbal remedies, also found in the global skin care market, have not yet been demonstrated to convey material beautification benefits to mammalian skin. Moreover, recent adverse findings regarding hormone replacement therapy have limited the viability of drug-based therapies in the beautification of mammalian skin. Although neutraceuticals (or functional foods) may prove effective in the context of skin beautification, considerable progress is required for the identification of safe and efficacious compounds with skin anti-aging benefits.

Thus, despite the immense efforts exerted by those skilled in the art, there remains a substantial and unmet need in the identification of age-defying and age-reversing products, and particularly compounds that are both efficacious and safe. For instance, estrogen provides unique skin benefits, but has numerous undesirable side effects due to its diversified role in other tissues - thereby limiting its use in a cosmetic skin care context. Estrogen effects are generally mediated by numerous estrogen receptors (*e*.*g*., ERα, ERβ, ERx) and estrogen receptors themselves are known to interact with other cellular proteins in a variety of tissues. This dilemma underscores the need to identify skin benefit agents that are specific to a cell or tissue type of interest. Contemporary advancements in genomic-proteomic and bioinformatic tools now facilitate the discovery of cell and/or tissue-type events via classical molecular genetic and biochemical approaches. Protein complexes generally control biological processes, and thus, the identification of skin-specific protein complexes and/or interaction partners encourages the discovery of skin anti-aging 'targets' and skin compounds with skin anti-aging benefits.

Hairless protein is a putative GATA family Zinc finger protein, for which several interaction partners exist. Indeed, the diversified interaction partners of HR continue to present a challenge and opportunity for pathway mapping and the discovery of certain skin and hair-enhancing products. Those of skill in the art have, to date, only identified a relationship between hairless protein and mammalian hair growth and maintenance. It has been recognized that individuals with point mutations in HR are hairless but are otherwise in good health. US Patent Number 6,348,348, issued February 19, 2002 to Thompson et al., discloses that HR interacts directly and specifically with thyroid hormone receptor (TR)-the same protein that regulates its expression. 6,348,348 further discloses a comparison of the amino acid sequences of the subject human HR sequence, a human sequence published by Ahmad et al. (Science, 279, 720-724, 1998); a human sequence published by Cichon et al. (Hum. Mol. Genet., 7, 1671-1679 and erratum at 1987-1988, 1998); a rat sequence published by Thompson (J. Neurosci., 16, 7832-7840, 1996); and a mouse sequence published by Cachon-Gonzalez et al. (Proc. Natl. Acad. Sci. USA, 91, 7717-7721, 1994). WO 99/38965 provides an isolated nucleic acid that encodes human hairless protein. Finally, commonly assigned US Patent Application serial number 10/452,858, filed on 02 June 2003 and entitled "Hairless Protein-Interacting Partner Complexes and Methods Thereof describes the use of hairless protein-interacting partner complexes for the inhibition or enhancement of hair growth. Although those of skill in the art have identified some interaction between HR and Thyroid Hormone receptors (TRs), never hereinbefore has an interaction between HR and other proteins been identified in Keratinocytes of mammalian skin. Accordingly, there remains a substantial and unmet need in the art to develop specific protein complexes that can be used to screen test agents, and thus, identify compounds that provide one or more beautification benefits for mammalian skin.

### Summary of the Invention

The present invention relates to hairless protein interacting partner complexes (HR-IP) for use in the screening and/or discovery of compounds adapted to provide material beautification and improvement benefits to mammalian skin. The present invention further relates to methods for identifying compounds exhibiting antagonist and/or agonist activity.

As will become apparent from the present disclosure, the present inventors employed a modified, yeast two-hybrid technology to identify the hairless protein interacting partner complexes disclosed herein, the interaction of which is believed to have many implications upon the condition of mammalian skin. The hairless protein interacting partner provided by the present invention is human Keratin 5, and include molecules involved in cell cycle, cell differentiation, transcription, nuclear transport, signal transduction, cellular integrity, and mitochondrial machinery.

Thus, the present invention, a composition comprising a mouse HRt protein-human interacting partner protein complex wherein the human interacting partner protein comprises Homo Spiens Keratin 5 is provided.

To reiterate, the aforementioned molecule are useful for screening test agents and identifying compounds that convey material beautification and improvement benefits to mammalian skin.

In another aspect of the present invention, a composition comprising a human interacting partner proteins encoded by a nucleic acid comprising SEQ ID NO 1, is disclosed.

In yet another aspect of the present invention, a method of assaying a test compound for agonist or antagonist activity in the beautification and/or improvement of mammalian skin is disclosed. Said method comprises the steps of a) measuring a level of interaction between mouse HRt protein and the human interacting partner in the absence of the test compound; b) measuring a level of interaction between mouse HR protein and the human interacting partner in the presence of the test compound; wherein when the level measured in step b) is greater than the level in step a), the test compound has agonist activity, and wherein when the level measured in step b) is less than the level in step a), the test compound has antagonist activity.

In another aspect of the present invention, compounds exhibiting agonist and antagonist activity, as hereinbefore defined, are disclosed and claimed. Said compounds may be identified in accordance with the present invention by employing the method of assaying a test compound, discussed *infra.* The agonist and antagonist compounds of the present invention may be delivered to a portion of mammalian skin for which the conveyance of material improvement and/or beautification benefits is desired. Said application and/or delivery may be achieved via a variety of ways, Including but not limited to, the incorporation of said compounds into a topical composition and/or the use of a pharmaceutical carrier for oral administration.

The compounds of the present invetion, whether exhibiting agonist or antagonist activity, may be formulated into a composition and/or incorporated into a product for the conveyance of material beautification and/or improvement benefits to mammalian skin.

### Detailed Description of the Invention

### Background and Definitions

The present invention relates to hairless protein interaction partner protein complex which comprises Homo Sapiens Keratin 5 (HR-IP),

A modified, improved yeast two-hybrid system (discussed *supra)* was used to identify protein interaction partners with hairless protein. The yeast two-hybrid system, in general, measures the association of two fusion proteins expressed in yeast. Interaction discovered in yeast is indicative of potential interaction that would occur under physiological conditions.

By "hairless protein (HR)" is meant herein the mouse hairless protein. By "truncated hairless protein (HRt)" is meant the sequence provided as SEQ ID NO 16, which is amino acid residues 490-1182 of the C-terminal portion of mouse HR protein. Derivatives, fragments, or analogs of HR known to one of skill in the art in light of the present disclosure are considered equivalents of HR. It should be noted and underscored that mouse HR is greater than 80% identical to human HR. Thus, the interacting partners provided herein are expected to interact with human hairless protein in the same manner as such interacting partners interact within mouse HR. Accordingly, an antagonist and/or agonist compound having activity for HRt is expected to further exhibit activity for HR. Antagonists or agonists of the present HRt-IP complexes are further expected to exhibit activity for human hairless protein interacting partner equivalents.

By "interacting partner (IP)," is meant a protein or nucleic acid that has sufficient binding affinity for HRt such that, when the IP and HRt are fused into separate constituents of a transcriptional regulator, the HRt-IP affinity is sufficient to allow reconstitution of the constituents of the transcriptional regulator - thereby allowing expression of reporter gene. The interaction may occur due to specific electrostatic, hydrophobic, hydrophilic, entropic or other interaction of certain portions of HRt with certain portions of IP to form a stable complex under conditions effective to promote the interaction. Interacting partners are identified by the data provided in Table 1. That is, the identity of the interacting partner was determined by homology searching using nucleotide sequence data from positive results in the yeast two-hybrid system. Derivatives, fragments, or analogs of interacting partners identified in Table 1 known to one of skill in the art in light of the present disclosure are considered equivalents of interacting partners, and thus, are well within the scope of the present disclosure.

By "HRt-IP complex," it is meant that at least one molecule of HRt associated with at least one molecule of interacting partner, under physiological conditions of ionic strength, temperature, pH and the like. The complex may be *in vivo* or *in vitro.*

In the yeast two-hybrid system, transcription of a reporter gene (*e*.*g*., LACZ, MEL1, HIS3, ADE1, URA3) is dependent upon reconstitution of a Gal 4 regulator by the interaction of two proteins, each fused to "half' of the regulator. When the two proteins have sufficient binding affinity to interact, the interaction results in the reconstitution of the GAL 4 regulator, which then activates transcription of the reporter gene(s). In the context of the present invention, "half" of the regulator is a DNA binding domain (BD), and "half" of the regulator is an activation domain (AD). When a "bait" protein is fused to the binding domain, and a "prey" protein is fused to the activator domain, or vice versa, sufficient binding affinity of the "bait" and "prey" proteins allow reconstitution of BD and AD to allow the reporter gene to be activated. Such an assay therefore is a test for affinity between the "bait" and "prey" proteins. The yeast two hybrid technology has been described in, for example, US Patent Number 5,986,055, issued November 16, 1999, to Yang, M., et al., incorporated herein by reference. The inventors of the present subject matter have employed a modified, yeast two hybrid technology, described in detail *infra.*

In the context of the present invention, the "bait" protein is a C-terminal portion of hairless protein of mouse (HRt) having amino acid residues 490 to 1182 (provided as SEQ ID NO:16, the nucleic acid sequence encoding amino acids 490 to 1182 is provided as SEQ ID NO:17) "Structure and Expression of the Hairless Gene of Mice," Begona, M., et al., J. Proc. Natl. Acad. Sci, USA 91:7717-7721, 1994) (GenBank accession no. Z32675). HR protein (aa residues 490 to 1182) contains the zinc finger domain (595-620) and three PEST domains previously identified to be potentially important for HR function. To reiterate, mouse HR very similar to human HR. Indeed, PEST sequences are often used to serve as signals for rapid protein degradation. The signals may be constitutive or conditional. The PEST-FIND computer program has been developed by M. Rechsteiner and S. W. Rogers (PEST sequences andregulation by proteolysis, TIBS 21, July 1996) to objectively determine whether a protein contains a PEST region. The associated algorithm, available in PC/GENE (OMIGA), defines a PEST sequence as a hydrophilic stretch of 12 or more aa residues containing at least one of each of P, E/D, and S/T in any order. PEST sequences must further be flanked by basic aa residues K, A or H, but basic residues are disallowed in the PEST sequence. PEST-FIND produces a score ranging from about +50 to -50. For classification purposes, a score of less than zero denotes a possible PEST region, but a score of greater than +5 denotes a high potential of the existence of a PEST region. For example, PEST Domains were located in the following regions of the HR protein (aa residues 490 to 1182): PEST Domain 12.26 between aa residues 522 and 546; PEST Domain 7.56 between aa residues 709 and 722; and PEST Domain 21.71 between aa residues 729 and 744.

In the context of the present invention, the "prey" proteins are encoded by a human Keratinocyte AD-cDNA library. When two complimentary mating types of yeast, one containing the BD-HRt fusion protein, and the other containing the AD-human cDNA protein, are mated, the progeny express each fusion protein. Those AD-proteins having sufficient affinity for HRt will bind HRt and allow the BD and AD to reconstitute to activate a reporter gene. Therefore, positive results from the two-hybrid system demonstrate affinity and interaction between the mouse HRt and a human Keratinocyte protein.

By an "antagonist" of HR-IP interaction, is meant an agent having inhibitory activity for the binding of the HR-IP complex. The binding may be inhibited by an effect on the interaction between HR and IP, or by an individual effect on HR or IP that affects the collective interaction between HR and IP.

By an "agonist" of HR-IP interaction, is meant an agent having enhancing or stimulatory activity for the binding of the HR-IP complex. The binding may be stimulated by an effect on the interaction between HR and IP or by an individual effect on HR or IP that affects the collective interaction between HR and IP. Identification of an antagonist or an agonist is made by allowing HR and IP to interact in the presence of a test agent. A decrease or increase in HR-IP interaction relative to the interaction in the absence of the test agent generally indicates that the test agent has an affect on the interacting pair as further described *infra.*

By "beautification and/or improvement benefits," it is meant that the hairless protein interacting partner complexes of the present invention may be used to screen and/or identify compounds having antagonist and agonist activity for binding of the HR-IP complex, that convey one or more of the following benefits to mammalian skin: Improved epidermal barrier function and hydration, cellulite reduction, enhanced barrier repair, prevention as well as reversal of skin wrinkling and hair growth retardation.

Since mouse HR is greater than 80% identical to human HR, the present inventors would expect with reasonable certainty that the human proteins that interact with mouse HRt provided by the present invention would also interact with human HRt. Similarly, test compounds that prove to be agonists or antagonists of a mouse HRt-human interacting partner interaction are expected by the present inventors to be agonists or antagonists of the human HRt-human interacting partner.

In HR-interacting partner protein compositions of the present invention, conservative amino acid substitutions (*e*.*g*. Glu/Asp, Val/Ile, Ser/Thr, Arg/Lys and Gin/Asn) would be considered equivalent since the chemical similarity of these pairs of amino acid residues would be expected to result in functional equivalency. Amino acid substitutions that conserve the biological function of the HR-interacting partner protein would conserve such properties as hydrophobicity, hydrophilicity, side-chain charge, or size. Functional equivalency is determined by the interaction of the equivalent pair as compared to the native pair. Included within the scope of the invention are complexes of HR-interacting protein fragments, derivatives, or analogs that are modified during or after translation, *e*.*g*., by glycosylation, acetylation, phosphorylation, amidation, fatty acylation, sulfation, derivatization by known protecting/blocking groups, proteolytic cleavage, linkage to an antibody molecule or other cellular ligand, or the like.

In the HR-interacting partner nucleic acid compositions of the present invention, a molecule that is the complement of a nucleic acid molecule that hybridizes to the interacting partner nucleic acid under stringent hybridization conditions would be considered equivalent since such a molecule is expected to result in functional equivalency as an interacting partner nucleic acid. Functional equivalency is determined by the interaction of the equivalent pair as compared to the native pair. Stringency conditions for hybridization depend upon a number of factors such as the length of hybridizing molecules, salt concentration, temperature, and the presence or absence of denaturing agents, for example. High stringency conditions may include hybridization at about 42°C and about 50% formamide, 0.1 mg/mL sheared salmon sperm DNA, 1% SDS, 2X SSC, 10% Dextran sulfate, a first wash at about 65°C, about 2X SSC, and 1% SDS, followed by a second wash at about 65°C and about 0.1X SSC. Alternatively, high stringency conditions may include hybridization at about 42°C and about 50% formamide, 0.1 mg/mL sheared salmon sperm DNA, 0.5% SDS, 5X SSPE, 1X Denhardt's, followed by two washes at room temperature and 2X SSC, 0.1% SDS, and two washes at between 55-60°C and 0.2X SSC, 0.1 % SDS.

HR-IP complexes may be used for the preparation of polyclonal or monoclonal antibodies, antibody fragments, humanized antibodies, single chain antibodies for affinity purification, detection and/or other functional studies. Methods for producing antibodies are well known in the art and can be applied to HR-IP complexes in light of the present disclosure. Antibodies having binding specificity for the HR portion or the IP portion of the HR-IP complex may be removed from an anti-HR-IP preparation by adsorption with HR or with the IP. Remaining antibodies will have specificity for the complex.

### Background on Mouse HR Mutations

The hairless (HR) gene is expressed in a large number of tissues, primarily the skin, and a mutation in the HR gene is responsible for the typical cutaneous phenotype of hairless mice. Mutant HR mouse strains show immune defects involving especially T cells and macrophages, as well as an age-related immunodeficiency and an accelerated atrophy of the thymus. (Ref: The thymus of the hairless rhino-j (hr/rh-j) mice. Jose et al. J Anat 2001 Apr;198(Pt 4):399-406
**Hairless mice (hr/hr):** The original hairless mutation was the first to be characterized at the molecular level. Murine leukemia virus pmv43 insertion in the intron 6 of HR gene causes aberrant splicing and a correctly spliced HR is expressed at approximately 5% of the normal level. Thus, this type of mouse expresses a small amount of the correct HR protein. (Ref: Role of endogenous retroviruses as mutagens: the hairless mutation of mice. Stoye etal. Cell 1988, 54:383-391).
**Rhino Yurlovo mice (hr^{rhY}):** A 13 bp insertion at nucleotide position 3520 in exon 16 of HR gene. This results in frame shift and premature termination at 264 bp downstream from the site of insertion in exon 17, and 139 bp upstream from the endogenous stop codon in exon 19. (Ref: Molecular basis for the rhino Yurlovo (hr^{rhY}) phenotype: Severe skin abnormalities and female reproductive defects associated with an insertion in the hairless gene. Panteleyev et al., Exp. Dermatol. 1998, 7:281-288)
**Rhino mice (hr^{rh-8j}):** A 2 bp substitution at nucleotide positions 1910 and 1911 of exon 4, resulting in a glutamine to histidine amino acid substitution at position 511 (Q511H) and a lysine to a nonsense codon at the adjacent amino acid 512 (K512X). Rhino is a functional knock-out of the HR gene. (Ref: Molecular basis for the rhino(hr^{rh-8j}) phenotype: A nonsense mutation in the mouse hairless gene. Ahmad et al., Genomics 1998, 53:383-386)
**Rhino mice (hr^{rhChr}):** C to T transition resulting in the conversion of an arginine residue (CGA) at amino acid position 467 (R467X) to a premature termination codon (TGA). (Ref: Molecular basis of a novel rhino (hr^{rhChr}) phenotype: a nonsense mutation in the mouse hairless gene. Ahmad et al. Exp. Dermatol. 1998, 7:298-301).
**Rhino mice (hr^{rh}/hr^{rh}):** Expresses a truncated version of HR (C-terminal amino acids 711-1189 are absent) due to premature stop in exon 6. (Ref: The hairless gene of the mouse: relationship of phenotypic effects with expression profile and genotype. Gonzalez et al. Dev Dyn. 1999 Oct; 216(2):113-26).

| **Mutation Spectrum in human HR causing congenital atrichias** | | | | | |
|---|---|---|---|---|---|
| Mutation | Location | Phenotype | Dermal cysts | Origin | Reference |
| p.R33X | Exon2 | APL | Yes | Mediterranean | Zlotogorski et al., (2002a) |
| c.1256delC | Exon 3 | AUC | Yes | Palestenian | Ahmad et al(1999a) |
| p.Q478X | Exon 4 | APL | Yes | Pakistani | Sprecher et al (1999b) |
| p.R613X | Exon 6 | APL | Yes | Japanese | Ahmad et al (1999b) |
| p.R620Q | Exon 6 | AUC | Yes | Irish | Ahmad et al (1998b) |
| p.C622G | Exon 6 | APL | Yes | Polish | Aitaet al (2000) |
| c.2001del CCAG | Exon 7 | APL | Yes | Mexican | Kruse et al (1999) |
| c.2147delC | Exon 9 | APL | Yes | Palestenian | Zlotogorski et al (1998) |
| c.2776 +1 G>A | Exon 12 | AUC | No | Omani | Cichon et al (1998) |
| p.N970S | Exon 14 | APL | Yes | Tyrolian | Kruse et al (1999) |
| p.D1012N | Exon 15 | AUC | Yes | Arab Israeli | Klein et al(2002) |
| p.T1022A | Exon 15 | AUC | Yes | Pakistani | Ahmad et al (1998a) |
| p.V1056M | Exon 16 | APL | Yes | Palestenian | Zlotogorski et al(2002b) |
| p.V1136D | Exon 18 | AUC | Yes | Pakistani | Cichon et al (1998) |
| c.de13434C | Exon 18 | APL | Yes | Arab Israeli | Sprecher et al (1999a) |

| | | | | | |
|---|---|---|---|---|---|
| Location of the mutation refers to GeneBank accession numbers AF039196 (nucleotide sequence) and NP_005135 (amino acid sequence) APL= Atrichia with papularlesions AUC=alopecia universalis congenita | | | | | |

References: Zlotogorski et al., (2002a), Clinical and molecular diagnostic criteria of congenital atrichia with papular lesions, J Invest Dermatol. 2002, 118:887-890. Ahmad et al(1999a), Genomic organization of the humanhairless gene (HR) and identification of a mutation underlying congenital atrichia in the Arab Palestinian family, Genomics 56, 141-148. Sprecher et al (1999b), Atrichia with popular lesions resulting from a nonsense mutation within the human hairless gene, J. Invest. Dermatol 113, 687-690. Ahmad et al (1999b), A homozygous nonsense mutation in thezinc-finger domain of the human hairless gene underlines congenital atrichia, J Invest Dermatol 113, 281-283. Ahmad et al (1998b), A miss sense mutation in the zinc-finger domain of the human hairless gene underlines congenital atrichia in a family of Irish travelers, Am J Hum Genet 63, 984-991. Aitaet al (2000), A novel missense mutation (C622G) in the zinc-finger domain of the human hairless gene associated with congenital atrichia and popular lesions, Exp Dermatol 9, 157-162. Kruse et al (1999), Hairless mutations in two kindreds with autosomal recessive popular atrichia, J Invest Dermatol 113, 954-959. Zlotogorski et al(1998), Congenital atrichia in five Arab Palestinian families resulting from a deletion mutation in the human hairless gene, Hum Genet. 1998,103:400-404. Cichon et al (1998), Cloning, genomicorganization, alternative transcripts and mutational analysis of the gene responsible for autosomal recessive universal congenital alopecia, Hum Mol Genet 7, 1671-1679. Kruse et al (1999), Hairless mutations in two kindreds with autosomal recessive popular tarichia, J Invest Dermatol 113, 954-959. Klein et al(2002), A novel missense mutation affecting the human hairless thyroid receptor interaction domain 2 causes congenital atrichia, J Invest Dermatol 119, 920-922. Ahmad et al (1998a), Alopecia universalis associated with mutation in the human hairless gene, Science 279, 720-724. Zlotogorski et al(2002b), Evidence for pseudodominant inheritance of atrichia with papular lesions, J Invest Dermatol 2002,118:881-886. Sprecher et al (1999a), Identification of a genetic defect in the hairless gene in atrichia with popular lesions: evidence for phenotypic heterogeneity among inheritedatrichia, Am J Hum Genet 64, 1323-1329, all of which are incorporated herein by reference.

### First Aspect: HRt Protein-Human Interacting Partner Protein Complexes

In accordance with a first aspect of the present invention, a composition comprising a mouse HRt protein-human interacting partner protein complex wherein the human interacting partner protein comprises Homo SApiens Keratin 5 is provided. The molecule disclosed herein has been identified employing a modified yeast two hybrid technology, described *infra.* Indeed, the aforementioned molecules is useful for screening test agents and identifying compounds that convey material beautification and/or improvement benefits to mammalian skin.

In another aspect of the present invention, human interacting partner proteins encoded by a nucleic acid comprising SEQ ID NO: 1, are provided. Said nucleic acid sequences are additionally useful for screening test agents and identifying compounds that convey material beautification and/or improvement benefits to mammalian, and particularly human skin. Compounds may be tested for agonist and/or antagonist activity toward the HR-IP complexes, including the molecules and nucleic acid sequences disclosed herein, in accordance with subsequent sections of the present disclosure.

### Second Aspect: Antagonist and/or Agonist Compounds of HR-IP Complexes

The human interacting partner protein described in the preceding section may be employed to screen antagonist and/or agonist compounds that are adapted to bind to HR-IP complexes. The interaction of said compound with the HR-IP complex facilitates their employment in compositions and products, consumer and otherwise, for the conveyance of one or more material beautification and/or improvement benefits to mammalian skin.

### Screening for Antagonists or Agonists of HR-IP Complexes

HR-IP complexes or nucleic acids encoding HR-IP complexes may be used to screen for compounds that bind to HR-IP complexes and thus, such compounds have use as agonists or antagonists of HR-IP complexes. The invention therefore provides assays to detect molecules that either enhance or inhibit interaction of HR and an interacting partner. Such molecules may be small molecules, peptides, modified peptides, aptamers, nucleic acids, or modified nucleic acids, for example. A molecule that modulates activity of HR-IP is identified by contacting a test molecule either with HR or with IP prior to contacting HR and IP together, or allowing a three way binding among HR, IP and the test molecule. Modulation of HR-IP interaction may be measured by measuring the stability of the complex, the affinity or binding of the complex, rate of formation of the complex, the amount of complex, or another function of the complex. An increase or decrease in any of these parameters relative to the parameter in the absence of the test molecule indicates that the test molecule is an agonist or an antagonist of the complex. Methods for identifying such agonists or antagonists may be carried out using the modified yeast two-hybrid assay described herein, or may be carried out *in vitro,* for example.

Test agents to be screened for antagonist or agonist activity may be provided as mixtures of specified compounds, or as compound libraries, peptide libraries, antisera, antisense nucleic acids, random or combinatorial libraries, chemically synthesized libraries, recombinant libraries, or *in vitro* translation-based libraries. Agents may be screened for activity as competitive or non-competitive inhibitors of HR-IP interaction. In particular, fragments or analogs of HR or the IP may be screened for such inhibitory activity. Agents may be screened for inhibition or enhancement of binding of HR and IP under aqueous or physiological binding conditions in which HR-IP binding occurs in the absence of the agent to be tested. Agents that lessen the binding are identified as antagonists of the complex, and agents that enhance the binding are identified as agonists of the complex.

Typical binding conditions are, for example, an aqueous solution of 10-250 mM NaCl, 5-50 mM Tris-HCl, pH 5-8, 0.5% Triton X-100. Metal chelators or divalent cations may be added to improve binding. Binding temperatures may include that of an ice bath up to 42 degrees C. The time of incubation is sufficient to allow binding equilibrium to occur. Methods known to one of skill in the art in light of the present disclosure may be used to assay for binding between HR and an interacting partner and a test agent, including use of detectable labels and quantification of complex formation.

A yeast two-hybrid assay system may be used to test an agent for antagonist or agonist activity for HR-IP binding similar to the assay system set forth by US Patent Number 5,986,055, issued November 16, 1999, for CDK2-interacting proteins, incorporated herein by reference. The two-hybrid assay is carried out as described in the present examples, for the exception that it is done in the presence of a test agent. An increase or decrease in reporter gene activity relative to the activity exhibited in the absence of the test agent indicates that the test agent has an effect on the interacting pair.

Components of such a system include a reconstitutable transcriptional regulator and a reporter gene. The reconstitutable transcriptional activator comprises a DNA binding domain (BD) and an activator domain (AD) that, when reconstituted, induces transcription of the reporter gene. The nucleotide sequence encoding HRt, fragment, derivative, or analog thereof is fused to either the DNA binding domain or the activator domain, and the nucleotide sequences encoding an interacting partner identified from the data of Table 1 is fused to the other of the BD or AD. The DNA binding domain can be any DNA binding domain, as long as it specifically recognizes a DNA sequence within a promoter active for a reporter gene. The activator domain has activity for the binding domain of the transcriptional regulator. The reporter gene is operably linked to a promoter that contains a binding site for the DNA binding domain. As for example, binding of BD-HR to AD-IP leads to reconstitution of the BD-AD transcriptional regulator that activates expression of the reporter gene. The activation of transcription of the reporter gene occurs intracellularly, e.g., in prokaryotic or eukaryotic cells, preferably in cell culture. The reporter gene encodes a detectable marker molecule that can give rise to a detectable signal, *e*.*g*. a fluorescent protein or a protein that can be readily visualized or that is recognizable by a specific antibody or an enzyme such as LacZ or α-galactosidase and is readily assayed.

Strains of yeast are chosen for selectable markers that confer ability to grow under conditions that do not support the growth of cells not expressing the selectable marker, *e*.*g*. the selectable marker is an enzyme that provides an essential nutrient and the cell in which the interaction assay occurs is deficient in the enzyme and the selection medium lacks such nutrient. The reporter gene is under the control of the native promoter that naturally contains a binding site for the DNA binding protein, or under the control of a heterologous or synthetic promoter.

Examples of transcriptional regulator proteins that have separable binding and transcriptional activation domains include, for example, the GAL4, the GCN4, and the ARD1 proteins of *S*. *cerevisiae* and the human estrogen receptor. The DNA binding domain and activation domain that are employed in the fusion proteins need not be from the same transcriptional regulator. For example, a GAL4 or a LEXA DNA binding domain is employed, or a GAL4 or herpes simplex virus VP16 activation domain is employed. In one embodiment, the yeast transcription factor GAL4 is reconstituted by the protein-protein interaction and the host strain is mutant for GAL4. Other embodiments are known to one of skill in the art in light of the present disclosure.

To facilitate isolation of the encoded interacting partners, the fusion constructs can further contain sequences encoding affinity tags such as glutathione-S-transferase or maltose-binding protein or an epitope of an HR or HRt antibody, for affinity purification for binding to the HR-IP. Where the IP is a nucleic acid, an affinity tag may be a hybridizing nucleotide sequence complementary to the nucleic acid IP.

The host cell in which the interaction assay occurs can be any cell, prokaryotic or eukaryotic, in which transcription of the reporter gene can occur and be detected, including but not limited to mammalian (e.g., monkey, chicken, mouse, rat, human, bovine), bacteria, and insect cells, and is preferably a yeast cell. Expression constructs encoding and capable of expressing the binding domain fusion proteins, the transcriptional activation domain fusion proteins, and the reporter gene product are provided within the host cell, by mating of cells containing the expression constructs, or by cell fusion, transformation, electroporation, or microinjection, for example. The host cell used should not express an endogenous transcription factor that binds to the same DNA site as that recognized by the DNA binding domain fusion population. In addition, the host cell is mutant or otherwise lacking in an endogenous, functional form of the reporter gene used in the assay.

Various vectors and host strains for expression of the two fusion protein populations in yeast are known and can be used (*see* US Patent Number 5,986,055, issued November 16, 1999). For example, yeast strains or derivative strains made therefrom, which can be used are N105, N106, N1051, N1061, and YULH, Y190: MATa, ura3-52, his3-200, lys2-801, ade2-101, trpI-901, leu2-3,112, gal4.DELTA., ga180.DELTA., cyh.sup.r 2, LYS2::GAL1.sub.UAS -HIS3.sub.TATA HIS3, URA3::GAL1.sub.UAS -GAL1.sub.TATA-lacZ. CG-1945: MATa, ura3-52, his3-200, lys2-801, ade2-101, trpl-901, leu2-3,112, gal4-542, gal80-538, cyh.sup.r 2, LYS2::GAL1.sub.UAS -HIS3.sub.TATA HIS3, URA3::GAL1.sub.UAS17mers(x3) -CYC1.sub.TATA -lacZ. Y187: MAT-.alpha., ura3-52, his3-200, ade2-101, trp1-901, leu2-3,112, gal4.DELTA., gal80.DELTA., URA3::GAL1.sub.UAS -GAL1.sub.TATA -lacZ. SFY526: MATa, ura3-52, his3-200, lys2-801, ade2-101, trp1-901, leu2-3,112, gal4-542, gal80-538, can.sup.r, URA3::GAL1-lacZ. HF7c: MATa, ura3-52, his3-200, lys2-801, ade2-101, trp1-901, leu2-3,112, gal4-542, gal80-538, LYS2::GAL1-HIS3. URA3::GAL1.sub.UAS17MERS(X3) -CYC1-IacZ.. YRG-2: MATa, ura3-52, his3-200, lys2-801, ade2-101, trp1-901, leu2-3,112, gal4-542, ga180-538 LYS2::GAL1.sub.UAS -GAL1.sub.TATA -HIS3, URA3::GAL1.sub.UAS17mers(x3) -CYC1-lacZ.

Plasmids are capable of autonomous replication in a host yeast cell and preferably are capable of being propagated in *E*. *coli.* The plasmid contains a promoter directing the transcription of the DNA binding or activation domain fusion genes, and a transcriptional termination signal. The plasmid also preferably contains a selectable marker gene, permitting selection of cells containing the plasmid. The plasmid can be single-copy or multi-copy. Single-copy yeast plasmids that have the yeast centromere may also be used to express the activation and DNA binding domain fusions. In another embodiment, the fusion constructs may be introduced directly into the yeast chromosome via homologous recombination using methods known to one of skill in the art in light of the present disclosure.

Plasmids encoding the separate fusion proteins can be introduced into a single host cell (e.g., a haploid yeast cell) containing one or more reporter genes, by co-transformation, to conduct the assay for HRt-IP interactions. Alternatively, the two fusion proteins are introduced into a single cell by mating (e.g. for yeast cells) or cell fusions (e.g., of mammalian cells). In a mating type assay, conjugation of haploid yeast cells of opposite mating type that have been transformed with a binding domain fusion expression construct and an activation domain fusion expression construct, respectively, delivers both constructs into the same diploid cell. The mating type of a yeast strain may be manipulated by transformation with the HO gene.

In one aspect, a yeast interaction mating assay is employed, using two different types of host cells, strain-types a and alpha, of the yeast *Saccharomyces cerevisiae*. One set of host cells, for example the a strain cells, contains fusions of the HRt nucleotide sequences with the DNA-binding domain of a transcriptional activator, such as GAL4. The hybrid proteins expressed in this set of host cells are capable of recognizing the DNA-binding site on the reporter gene. The second set of yeast host cells, for example alpha strain calls, contains nucleotide sequences as identified by the data provided in Table 1 fused to the activation domain of a transcriptional activator. In one embodiment, the fusion protein constructs are introduced into the host cell as a set of plasmids.

Bacteriophage vectors may be used to express the DNA binding domain and/or activation domain fusion proteins. Libraries can generally be prepared faster and more easily from bacteriophage vectors than from plasmid vectors.

Reporter genes include URA3, HIS3, lacZ, MEL1, GFP, luciferase, LEU2, LYS2, ADE2, TRP1, CAN1, CYH2, GUS, CUP1, or CAT, for example. Expression of LEU2, LYS2, ADE2 and TRP1 are detected by growth in a specific defined media; GUS, lacZ, MEL1, and CAT can be monitored by well known enzyme assays; and CAN1 and CYH2 are detected by selection in the presence of canavanine and cycloheximide. The natural fluorescence of GFP is detected. In another embodiment, transcription of the reporter gene is detected by a linked replication assay known to those of skill in the art in light of the present disclosure. In another embodiment, the expression of a reporter gene is detected by immunoassay. Activity of the reporter gene lacZ is monitored by measuring a detectable signal, such as X-gal (5-bromo-4-chloro-3-indolyl-.alpha.-D-galactoside).

False positives arising from transcriptional activation by the DNA binding domain fusion proteins in the absence of a transcriptional activator domain fusion protein are prevented or reduced by negative selection.

In one embodiment of the invention, the DNA sequences encoding the pairs of interacting partners are isolated by amplification in separate respective reactions. Amplification is carried out by polymerase chain reaction known by one of skill in the art using pairs of oligonucleotide primers specific for either the DNA-binding domain hybrids or the activation domain hybrids. Other amplification methods known in the art can be used, including but not limited to ligase chain reaction, use of Q beta. replicase, and the like.

### DUALhybrid Screen II Package B Screening

The DUALhybrid Screen II Package B System, available from Dualsystems Biotech AG of Zurich, Switzerland, was used to identify interaction partners for HR in human keratinocytes. A full description of this screening method is available from Dualsystems Biotech, available by submitting an electronic mail request at info@dualsystems.com or via the Internet at http://www.dualsystems.com. This screening method generally involves the steps of constructing and verifying a bait vector; transforming a large-scale library and selecting positive clones; isolating and restricting the analysis of positive clones; retransforming and testing clones for bait dependency; and sequencing bait dependent clones. Information on the precise vector library (pACT2), as well as the complete nucleotide sequence employed is available at the web site of Clontech, http://www.clontech.com.

### Purification of HR-IP complexes

HR-IP complexes may be isolated and purified by standard methods known in the art from natural sources or recombinant host cells expressing the complexes, including but not limited to column chromatography (*e*.*g*., ion exchange, affinity, gel exclusion, reverse-phase high pressure, fast protein liquid, and the like), differential centrifugation, differential solubility, or by other standard techniques used in purification of protein complexes or protein-nucleic acid complexes. The amino acid sequence of an interacting protein can be deduced from the nucleic acid sequence of the chimeric gene from which it was encoded. As a result, the protein or a derivative thereof can be synthesized by standard chemical or recombinant methods known in the art.

### PREPARATIVE EXAMPLES

### EXAMPLE 1 - Interaction Partners (IP's) of Hairless Protein

The present example provides for Hairless Protein (HR) interacting partners (HR-IP) using the yeast two-hybrid technology. HR cDNA corresponding to the C-terminal half of HR protein (aa residues 490 to 1182, hereinafter HRt) is cloned into DUALhybrid bait vector so as to express Lex A DNA BD-HRt in yeast. Yeast cells expressing BD-HRt are transformed with human keratinocyte cDNA library in the activation domain (AD) vector. The resultant interacting partners provided by the present invention are listed in Table 1 that include molecules involved in cell cycle, cell differentiation, transcription, nuclear transport, signal transduction, cellular integrity, and mitochondrial machinery. Thus, HRt (and HR) appears to be a well-connected, multi-functional protein.

### Materials

A mouse early anagen cDNA library is constructed by personnel at Procter & Gamble using standard techniques. *E. coli* expression plasmid pET32a (Catalog No. 69015-3; component plasmid of Novagen's pET prokaryotic expression system) and *E. coli* strain BL21(DE3) competent cells (Catalog No. 69387-3) are purchased from Novagen (Madison, WI). Custom designed oligonucleotides are synthesized by personnel at Procter & Gamble using standard techniques or made by BD Biosciences Clontech (PaloAlto, CA). DNA sequence determination is performed by personnel at Procter & Gamble using standard techniques or contracted out to Seqwright Inc (Houston, TX). DNA sequence analyses and data base searches for homology are performed using SeqWeb version 1.2 (in conjunction with Wisconsin Package Version 10.1). Various microbiological media are purchased from BD Biosciences Clontech (PaloAlto, CA) and DIFCO Becton Dickinson Microbiology Systems (Sparks, MD). Various pre-made media plates for cultivation of *E*. *coli* and yeast are purchased from Gibson Laboratories (Lexington, KY). Matchmaker two-hybrid System 3 (Catalog No. K1612-1), pre-transformed human brain Matchmaker cDNA library in pACT2 vector (Catalog No.HY4004AH, Lot No.9070550), Advantage 2 PCR kit (catalog No. K1910-1), and Matchmaker AD LD-insert screening amplimer sets (Catalog No. 9103-1), are purchased from BD Biosciences Clontech (PaloAlto, CA). Male mice (C3H strain) are purchased from Harlan Labs (Indianapolis, IN). NAIR lotion hair remover (4 minute formula) is purchased from a local Walgreens store. Dolgel Gel (5% ibuprofen) is purchased from Life Extension International Center Inc. (Coral Gables, FL). Restriction enzymes, T4 DNA ligase, molecular weight markers, agarose, and other routine molecular biology reagents are purchased from Life Technologies (Gaithersburg, MD).

### Cloning and characterization of mouse Hairless (HR) cDNA

Oligonucleotides are designed to PCR-amplify the desired portion of mouse HR cDNA, based on the published sequence for mouse HR "Structure and Expression of the Hairless Gene of Mice," Begona, M., et al., J. Proc. Natl. Acad. Sci, USA 91:7717-7721, 1994) (GenBank accession no. Z32675). The oligonucleotides are also designed to contain restriction enzyme recognition sites for *Eco* RI, *Xba* I, *Not* I and *Bam* HI restriction enzymes for subsequent manipulation of the PCR product. In particular, the restriction enzyme sites for *Eco* RI and *Not* I (noted as underlined and italicized in the oligonucleotides shown below) are particularly useful for subsequent cloning and manipulation. The forward and reverse oligonucleotide primer pairs 5'-CCG GAA TTC GTC ACC CAG TGC CAA AGC TGT (SEQ ID NO:100) and 5'-CGG GAT CCT CTA GA*G CGG CCG CTT* ATT ATT TAG CTT CCT GTA ACG CCCC (SEQ ID NO:101) are used to PCR amplify HR cDNA corresponding to nucleotides 1845-3923 of HR from mouse early anagen cDNA library. PCR amplification is performed using the standard PCR protocol supplied with the Advantage 2 PCR kit. The PCR product is analyzed by agarose gel electrophoresis and is found to have the expected size (approximately 2 Kb). The PCR product is excised with *Eco* RI and *Not* I restriction enzymes. The restricted product is gel-purified and inserted into *Eco* RI and *Not* I sites of pET32a, such that the insert makes an in-frame fusion with upstream sequences. The resulting plasmid is designated pET32a-HRt. This plasmid is used for both nucleotide sequence confirmation as well as for protein expression in *E*. *coli*. The nucleotide sequence reveals changes at three positions (nucleotide position 2166, change from C to T causing a codon change from AGC to AGT; nucleotide position 2611, change from C to T causing a codon change from GAC to GAT; nucleotide position 2916, change from T to C causing a codon change from CCT to CCC) from the published sequence (SEQ ID NO:102). Amino acid number 1 of SEQ ID NO:103 corresponds to amino acid residue 490 of the C-terminal portion of HR protein. This truncated HR (HRt) cDNA corresponds to amino acid residues 490-1182 of the C-terminal portion of HR protein.

However, none of the above-cited changes results in an amino acid change. In essence the HRt cloned herein is predicted to code for a protein product identical to that of mouse HRt. The zinc finger region (amino acids 595-620) and three PEST domains are present in the 490-1182 amino acid portion of HR. The PEST domains (region 522-546 having a score of 12.26, region 709-722 having a score of 7.56, and region 729-744 having a score of 21.71) are expected by the present inventors to be important in HR function. Mouse HR is greater than 80% identical to human HR. PEST sequences are often found to serve as signals for rapid protein degradation. PEST sequences are constitutive or conditional signals. Constitutive means "as such," while conditional means "upon modification" (such as phosphorylation, *cis-trans* isomerisation induced by light, ligand binding, etc.). To objectively determine whether a protein contains a PEST region, a computer program called PEST-FIND has been developed by M. Rechsteiner and S. W. Rogers (PEST Sequences and Regulation by Proteolysis, TIBS 21, July 1996). The algorithm, which is available in PC/GENE (OMIGA), defines a PEST sequence as a hydrophilic stretch of 12 or more amino acid residues containing at least one each of Pro, Glu/Asp, and Ser/Thr in any order or combination. They also have to be flanked by basic amino acid residues Lys, Ala, or His, but basic residues are disallowed within the PEST sequence. PEST-FIND produces a score ranging from about +50 to -50. By definition, a score >0 denotes a possible PEST region, but a value > +5 indicates a highly probable PEST region. The PEST regions noted herein also have numerous potential protein kinase C and casein kinase II phosphorylation sites. The PEST scores cited supra are among the best ever reported in the literature.

pET32a-HRt is also an inducible *E. coli* expression plasmid. Protein expression is studied using the standard protocol for *E*. *coli* expression as also provided by the supplier. SDS-PAGE (10-20%) analysis of total protein isolated from quadruplicate *E*. *coli* BL21(DE3)/pET32a-HRt clones induced for protein expression reveals a high level of expression of protein product of the expected size for a HRt fusion protein just after 2h of induction. Control strain *E. coli* BL21 (DE3)/ pET32a does not show induction of any protein expression at the high molecular weight region as compared to the *E*. *coli* BL21 (DE3)/ pET32a-HRt clones. This study confirms expression of protein product from the HRt insert. The protein product is not recoverable under non-denaturing conditions.

### Cloning of HRt into GAL4 binding domain (BD) yeast two-hybrid (Y2H) vector

The HRt insert from pET32a-HRt is excised as an *Eco* RI-*Xho* I fragment (there is a unique *Xho* I site just adjacent to HRt insert) and inserted at the *Eco* RI-*Sal* I sites of Y2H-BD vector pGBKT7 (Catalog No. K1612-B; a component of Matchmaker two-hybrid System 3 (Catalog No. K1612-1) BD Biosciences Clontech, Palo Alto, CA) to obtain pGBKT7-HRt in which HRt is fused in-frame to GAL4 DNA BD (this plasmid is also designated GAL4 BD-HRt). This plasmid was combined with AD-IP in *S. cerevisiae* AH109 strain screen for agonists and antagonists of HRt-IP interaction (see Example 2 and 3). However, to screen for IP in the first place, HRt from GAL BD-HRt was excised out and inserted into DUALhybrid bait vector (this plasmid is also designated LexA BD-HRt).

### Human Keratinocyte cDNA Library Screen for HRt interaction

Clontech Human Keratinocyte GAL4 cDNA library (complexity 2.5X10⁶) in pACT2 vector was used used to screen for HRt interacting proteins. A Leu⁻, Trp⁻ yeast strain with HIS3 and LACZ reporters for yeasts two hybrid interaction (strain L41) was first transformed with LexA BD-HRt palsmid) for Trp⁺ phenotype. The resulting L41/LexA BD-HR transformant was screened to ensure expression of LexA BD-HRt protein and also for lack of auto activation of the reporter genes HIS3 and LACZ. This strain was transformed with Keratinocyte AD-cDNA library and plated on Trp⁻Leu⁻His⁻ plate to select for clones with positive interaction. Colonies that appeared on selective plates were subsequently tested for LACZ expression on X-GAL plates. A total of 90 colonies were identified. AD-plasmid was rescued from the 90 clones and retested for HRt bait dependance. Fifty four isolates were confirmed as positive. The positive clones are expected to contain an AD-IP (interacting partner) that interacts with BD-HRt. To characterize IP, the sequence of IP is determined and matched to the human genome database DNA sequence analysis and data base search for homology are performed using SeqWeb version 1.2 (in conjunction with Wisconsin Package Version 10.1).

Several human interacting partners that interact with HRt are identified as listed in Table 1. Proteins having SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO:13, SEQ ID NO:14 and SEQ ID NO: 15 appear to be new proteins. The present invention concerns interacting partner proteins encoded by SEQ ID NO:1; these are employed in the exemplified assaying of test compounds.

**Table 1**

| *BD-HRt(mouse) interaction with human keratinocyte c-DNA library in AD-Vector* **Represents AD-HRt(mouse) interaction with BD-VDR(mouse)* | | | |
|---|---|---|---|
| | | | SEQ ID NO: |
| **GenBankAcc #** | **Clone Identity** | Hits | |
| BC024292 | Homo Sapiens Keratin 5 | 1 | 1 |
| BC007790 | Homo Sapiens Ubiquitous Receptor | 14 | 2 |
| AF167160 | Homo Sapiens Protein Inhibitor of Activated STAT-1 (PIAS 1) | 1 | 3 |
| BC001765 | Homo Sapiens Similar to Stromal Antigen 2 | 4 | 4 |
| XM_113678 | Homo Sapiens Nucleoporin 160 Kda | 3 | 5 |
| M57707 | Homo Sapiens Retinoic Acid Receptor Gamma-1 | 1 | 6 |
| BC000261 | Homo Sapiens Thyroid Hormone Receptor Alpha | 7 | 7 |
| BC001275 | Homo Sapiens Annexin A1 (Lipocortin 1 or P35) | 1 | 8 |
| AF054589 | Homo Sapiens HIC Protein Isoform P32 and Isoform 40 | 1 | 9 |
| | Homo Sapiens Insulin-like Growth Factor Binding Domain | | |
| BC011708 | Protein 6 | 1 | 10 |
| | Homo Sapiens Inner Membrane Protein, Mitochondrial | | |
| BC002412 | (Mitofilin) | 3 | 11 |
| | Homo Saplens Endoplasmic reticulum thioredoxin superfamily | | |
| BC001493 | member | 1 | 12 |
| BC030556 | Homo Sapiens Protein Inhibitor of Activated STAT-3 (PIAS3) | 1 | 13 |
| BC011819 | Homo Sapiens DEAD (Asp-Glu-Ala-Asp) box polypeptide 3 | 2 | 14 |
| BC015970 | Homo Sapiens Dpy-30 Like Protein (Dev. Nuclear Protein) | 1 | 15 |
| BC006716 | Mus Musculus Vitamin D Receptor (VDR) | 1 | 16 |

### EXAMPLE 2 - Screen for compounds that disrupt BD-HRt><AD-IP interaction in yeast strain

AH109 was used for compound screening. AH109 (a component of the Matchmaker two-hybrid System 3 (Catalog No. K1612-1) BD Biosciences Clontech, Palo Alto, CA) is a desirable yeast reporter strain for Y2H interaction. It contains *ADE2*, *HIS3*, *lacZ* and *MEL1* reporter genes, each of which uses a distinct GAL4-responsive promoter. The various Y2H recombinant plasmids are introduced into the yeast strain AH109 by using a lithium acetate-mediated yeast transformation protocol as provided by the supplier of the Y2H system (BD Biosciences Clontech, PaloAlto, CA). Transformants for combined AD and BD plasmids are selected based on their ability to grow in Leu and Trp double drop out medium (DDO).

Interaction between AD and BD fusion proteins (Y2H interaction) will bring the AD and BD domains of GAL 4 protein close to each other and thus reconstitute a functional GAL 4 protein. This will allow transformants to grow on Leu, Trp, His, Ade quadruple drop out medium (QDO). Growth rate on QDO, as well as a measure of MEL1 (alpha-galactosidase) or lacZ (beta-galactosidase) activities will give a more or less quantitative measure of avidity of Y2H interaction.

Growth inhibition of AH109 (BD-HRt><AD-IP) strain in Leu, Trp, His, Ade QDO, but not on Leu, Trp DDO is used as an initial screen for compounds for disruption of HRt-IP interaction. A diverse library of compounds is screened. Microtiter plates with 200 µl of QDO and DDO media seeded with freshly cultured yeast cells at 0.01-0.03 optical density at 650 nm (OD650) are treated with various test compounds. Stock solutions of test compounds at 20 mM are made in DMSO and tested at 400 µM final concentration. The plates are incubated without shaking at 25°C. OD650 is first measured after addition of compounds and recorded for the next 8-10 days. Wells with no compound added (positive control) are expected to reach saturation growth (- 0.7 within 3 days of incubation). Wells with only media and no cells inoculated are expected to remain at the initial OD650 value (∼0.03) over the entire period of the assay. Positive compounds from this assay are tested against an unrelated Y2H interaction (p53><T antigen) assay. pGBKT7-53 and pGADT7-T plasmids were used to establish an unrelated (to HRt) Y2H interaction in which p53 protein interacts with T antigen. These plasmids are part of the Matchmaker two-Hybrid system 3(catalog # K1612-1, BD Biosciences Clontech, Palo Alto, CA).

### EXAMPLE 3 - Screen for compounds that enhance or disrupt BD-HRt><AD-IP interaction

Increase or decrease in alpha-galactosidase activity of AH109 (BD-HRt><AD-IP) strain grown in Leu, Trp, His TDO in the presence of various test compounds is used to screen for agonists or antagonists of BD-HRt><AD-IP interaction. A diverse library of compounds is screened.
Microtiter plates with TDO media seeded with freshly cultured yeast cells at 0.05 optical density at 650 nm (OD650) are treated with various test compounds at 100-400 uM or vehicle (DMSO) control. Stock solutions of test compounds at 10 - 20 mM are made in DMSO and tested at 100 - 400 µM final concentration. The final assay mixture comprising of cells, TDO and compound (or DMSO alone) is adjusted to be 100 ul and the amount of DMSO is maintained at or below 2%. The plates are incubated for 3-4 days without shaking at 25°C. Plates are briefly agitated to obtain uniform suspension of cells prior to addition of assay buffer. Assay of reporter enzyme (alpha-galactosidase) activity is performed by incubating for 2-5 hours at 30 degree Centigrade with 100 ul assay buffer [acetate buffer (0.2M, pH 4.5) containing 0.025M para-nitropnenyl alpha galacto pyranoside (PNPG)]. The incubation time can be reduced or increased depending on the extent of alpha galactosidase activity for a given BD-HRt><AD-IP pair. Plates are briefly agitated to obtain uniform suspension of cells and absorbance at both 650 nM (OD650)_{ref} and 410 nm (00410)_{ref} are measured. OD650_{ref} serves as a reference value for cell density and 00410_{ref} serves as reference value prior to yellow color development for product (p-nitro phenol) generated in the assay. For color development 100 ul of 0.1M sodium carbonate solution is added and after standing for at least 1 minute, OD410ₑₓₚ is measured. Relative alpha-galactosidase activity is estimated using the following relation:
Relative activity = [OD410ₑₓₚ-OD410_{ref}x0.67]/OD650_{ref}
Positive compounds from this assay are tested against an unrelated Y2H interaction (p53><T antigen) assay to ensure that the compounds are specific to BD-HRt><AD-IP interaction.

### EXAMPLE 4 - Screen for interaction with AD-HRt

We have observed that, with certain IPs such as Thyroid hormone receptor (TR), the interaction is much stronger in the AD-HRt><BD-TR configuration than in the BD-HRt><AD-TR configuration. Based on this observation, we suspect that some of the IPs might have gone undetected in the BD-HRt><AD-cDNA library screen and additional IPs for HRt can be detected by also using a AD-HRt><BD-cDNA library screen.

## Claims

1. A composition comprising a mouse HRt protein-human interacting partner protein complex wherein the human interacting partner protein comprises Homo Sapiens Keratin 5.

2. A composition comprising a mouse HRt protein-human interacting partner protein complex, wherein said human interacting partner is encoded by a nucleic acid comprising SEQ ID NO: 1.

3. A method of assaying a test compound for agonist or antagonist activity for the composition of claim 1 or claim 2, comprising:
a) measuring a level of interaction between mouse HRt protein and the human interacting partner protein(Homo Sapiens Keratin 5) in the absence of the test compound;
b) measuring a level of interaction between mouse HRt protein and the human interacting partner protein(Homo Sapiens Keratin 5) in the presence of the test compound;
wherein when the level measured in step b) is greater than the level in step a), the test compound has agonist activity, and wherein when the level measured in step b) is less than the level in step a), the test compound has antagonist activity.

## Patentansprüche

1. Zusammensetzung, einen Maus-HRt-Protein-/Mensch-Interaktionspartnerprotein-Komplex aufweisend, wobei das Mensch-Interaktionspartnerprotein Homo-sapiens-Keratin 5 umfasst.

2. Zusammensetzung, einen Maus-HRt-Protein-/Mensch-Interaktionspartnerprotein-Komplex aufweisend, wobei der Mensch-Interaktionspartner von einer Nukleinsäure kodiert ist, die SEQ ID NO:1 umfasst.

3. Verfahren zum Analysieren einer Testverbindung auf eine agonistische oder antagonistische Aktivität für die Zusammensetzung nach Anspruch 1 oder Anspruch 2, umfassend:
a) Messen eines Interaktionsgrades zwischen Maus-HRt-Protein und dem Mensch-Interaktionspartnerprotein Homo-sapiens-Keratin 5 in Abwesenheit der Testverbindung;
a) Messen eines Interaktionsgrades zwischen Maus-HRt-Protein und dem Mensch-Interaktionspartnerprotein Homo-sapiens-Keratin 5 in Anwesenheit der Testverbindung;
wobei, wenn der in Schritt b) gemessene Grad höher ist als der in Schritt a) gemessene Grad, die Testverbindung eine agonistische Aktivität aufweist und wobei, wenn der in Schritt b) gemessene Grad niedriger ist als der in Schritt a) gemessene Grad, die Testverbindung eine antagonistische Aktivität aufweist.

## Revendications

1. Composition comprenant un complexe protéine HRt de souris-protéine partenaire interactive humaine dans laquelle la protéine partenaire interactive humaine comprend de la Kératine 5 d'Homo Sapiens.

2. Composition comprenant un complexe protéine HRt de souris-protéine partenaire interactive humaine, dans laquelle ledit partenaire interactif humain est codé par un acide nucléique comprenant SEQ ID No. : 1.

3. Procédé de dosage d'un composé de test pour une activité agoniste ou antagoniste pour la composition selon la revendication 1 ou la revendication 2, comprenant :
a) la mesure d'un niveau d'interaction entre la protéine HRt de souris et la protéine partenaire interactive humaine Kératine 5 d'Homo Sapiens en l'absence du composé de test ;
b) la mesure d'un niveau d'interaction entre la protéine HRt de souris et la protéine partenaire interactive humaine Kératine 5 d'Homo Sapiens en présence du composé de test ;
dans lequel lorsque le niveau mesuré dans l'étape b) est supérieur au niveau dans l'étape a), le composé de test a une activité agoniste, et dans lequel lorsque le niveau mesuré dans l'étape b) est inférieur au niveau dans l'étape a), le composé de test a une activité antagoniste.
